# EUROPEAN PATENT APPLICATION

(11) **EP 2 468 741 A1**
(43) Date of publication of application: **27.06.2012**
(21) Application number: 10474003.0
(22) Date of filing: 16.12.2010
(51) Int. Cl.: C07D 311/62, C07D 493/04, A61K 31/352, A61K 31/357, A61P 29/00

(54) **Novel quercetin derivatives, their preparation, pharmaceutical compositions containing them and their use**

(71) Applicant: Bel/Novamann International s.r.o., 811 09 Bratislava (SK)
(72) Inventor: Veverková, Eva, 841 08 Bratislava (SK); Veverka, Miroslav, 841 08 Bratislava (SK); Svajdlenka, Emil, 763 21 Slavicin (CZ); Ratkovska, L'ubica, 920 01 Hlohovec (SK); Vodny, Stefan, 841 02 Bratislava (SK)
(74) Representative: Zakova, Anna

(57) **Abstract**

The invention relates to novel quercetin derivatives and pharmaceutically acceptable salts, hydrates, and solvates and dimers thereof; a process for the preparation of the novel quercetin derivatives and pharmaceutically acceptable salts, hydrates, and solvates and dimers thereof; and pharmaceutical compositions comprising the same that are useful for the treatment of various disorders. The quercetin derivatives are shown by formula I and II.

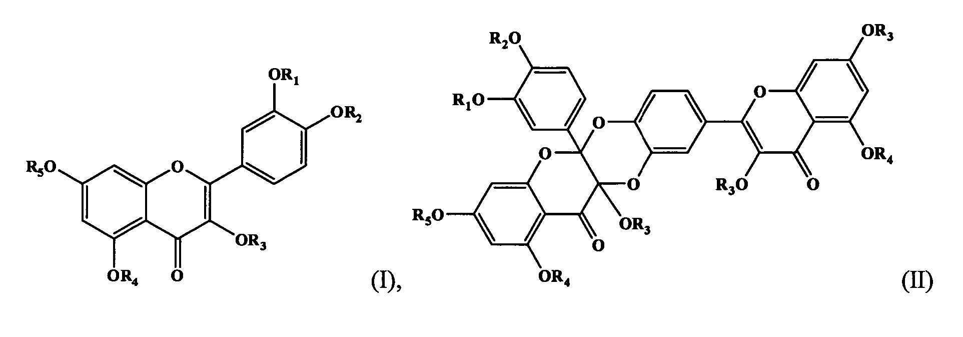

## Description

### FIELD OF THE INVENTION

The present invention relates to novel quercetin derivatives of formula (I) and pharmaceutically acceptable salts, hydrates, and solvates thereof; a process for the preparation of the novel quercetin derivatives of formula (I), and pharmaceutically acceptable salts, hydrates, and solvates thereof; and pharmaceutical compositions comprising the same that are useful for the treatment of various disorders.

### BACKGROUND OF THE INVENTION

Quercetin is the aglycon of quercitrin, rutin, and of other glycosides. It is widely distributed in the plant, e.g. in rinds and barks, in clover blossoms and in ragweed pollen. Being a polyphenols natural organic compound, it is one of the large numbers of plant pigments called flavonoids.

Due to its significant antioxidant capacity as well as several other biological activities, such as anti-diabetic, anti-inflammatory, coronary vasorelaxation properties, natural antihistamine, antiviral, enhancement of the immune system and others the quercetin molecule is promising as a new entity for treatment of various diseases.

The international publication No. WO 01/21164 describes use of quercetin derivatives for treating or preventing mycobacterium infections in immunocomprised patients, particularly HIV infected patients.

Studies have shown that quercetin exhibits anticancer effects (e.g. Annals of Oncology, 2001, 12, 245-248; J. Med. Chem., 2005, 48 (8), 2790-2804).

Documents US 3,420,815; US 4,202,815; US 5,955,100 and US 6,258,840 describes various chemical modifications of quercetin at the positions 3, 5, 7, 3' and 4' from the perspective of better transport through biological barriers.

Document US 6,235,294 describes flavonoid esters useful for pharmaceutical or dietetic compositions.

Document US 2002/0106338 describes a galenic formulation comprising flavonoid esters of 2-hydroxybenzoic acid and 4-methoxycinnamic acid.

The clinical developments of quercetin have been limited owing to its low water solubility and a limited solubility in pharmaceutically acceptable solvents.

In view of the above, there have been efforts to produce derivatives of Quercetin having improved aqueous solubility or solubility in pharmaceutically acceptable solvents which also, would be more soluble for use. It is important to modify selectively the hydroxyls group, which are different from the chemical reactivity point of view. Thus, regioselective O-derivatization of quercetine *via* ester intermediate (Molecules 2010,15, 4722) or protection of selective hydroxyl group *via* benzyl or diphenylmethane group (Tetrahedron 2002,58,10001) are applied.

Dimer of quercetin and various oxidized flavonoid derivatives were generated from quercetin with 2,2'-azobis-isobutyronitrile and isolated by chromatography (J. Agric Food Chem. 2002, 50, 4357) or isolated from *Allium cepa* (Chem. Nat. Compds. 2008,44 (4) 427). However their biological and pharmalogical data are limited owing to its low content in raw material (Chem. Nat. Compds. 2007,43 (3) UDC547.972). A naturally occurring quercetin heterodimer was isolated and its *in vitro* antioxidation properties were evaluated (Food Res. Int. 2007, 40 (1), 7). Oxidation of quercetin with K₃[Fe(CN)₆] give 9% yield of quercetine dimer and triquercetine structure in 7% yield (Chem. Nat. Compds. 2008, 44 (4), 427). Thus the accessability of quercetin heterodimer limited the studies of activities and preparation of new derivatives.

There exists a need, for further structural modifications in quercetin skeleton to obtain more potent biochemical compounds. In their endeavours to find novel quercetin derivatives, which are not only potent, therapeutically but also clinically acceptable, the present inventors have synthesized a number of quercetin derivatives of formula (I). The active compound compositions are effective for the prevention and treatment of photodermatitis, inflammation and allergy.

### OBJECT OF THE PRESENT INVENTION

An object of the present invention is to provide novel quercetin derivatives for the treatment of various disorders.

Another object of the present invention is to provide processes for preparation of novel quercetin derivatives.

A further object of the present invention is to provide pharmaceutical compositions comprising novel quercetin derivatives for the treatment of various disorders including photodermatitis, inflammation and allergy.

Still further object of the present invention is to provide a method of treatment of various disorders including photodermatitis, inflammation and allergy through administration of novel quercetin derivatives.

### SUMMARY OF THE INVENTION

In one aspect, the present invention provides novel 3,5,7,3',4' substituted flavonoid derivatives also known as quercetin derivatives of formula (I) and pharmaceutically acceptable salts, hydrates, and solvates thereof, 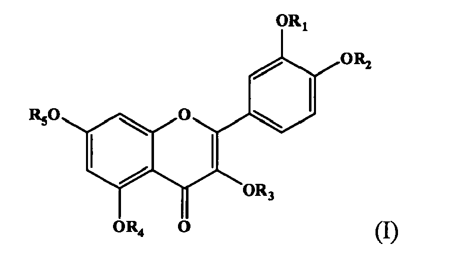 in which R₁ to R₅, identical or different, are independently selected from H, benzyl, 3-(2,6-dimethyl-morpholin-4-yl)-2-hydroxy-1-propyl, or a group R₆-(CH=CH)ₙ-CO- wherein R₆ is linear or branched substituted or unsubstituted alkyl C₁ to C₁₆ or alkenyl C₂ to C₁₆, cyclic alkyl radical having from 5 to 6 carbon atoms, wherein cyclic alkyl radical is independently mono-, di-, tri, tetra substituted in any position on the ring with OH, O-alkyl having saturated or unsaturated, linear, branched or cyclic hydrocarbon chains having from 1 to 10 carbon atoms, acetyl, or combination thereof, 1-adamantyl, aryl, wherein aryl is independently mono-, di-, tri substituted in any position on the ring with OH, O-alkyl having saturated or unsaturated, linear, branched or cyclic hydrocarbon chains having from 1 to 10 carbon atoms, acetyl, or combination thereof and n is 0 or 1; with the proviso that if in the group R₆-(CH=CH)ₙ-CO- substituent R₆ is phenyl and n is 0, hydroxyl group is not in ortho position or if R₆ is phenyl and n is 1, methoxy group is not in para position; and
a dimer of the compound of general formula (I) wherein R₁ to R₅ have the meanings given for general formula (I); and the isomers of the compound of general formula (I) and its dimer.

Another object of the present invention is to provide the dimer of the compound of structural chemical formula (I) having general formula (II) 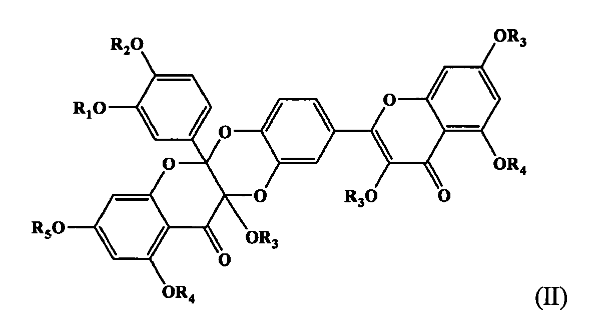 wherein R₁ to R₅ have the meanings given in formula I, with the proviso that at least one of the substituent R₁ to R₅ is hydrogen atom.

In another aspect, the present invention provides a process for preparation of the novel quercetin derivatives of formula (I), and pharmaceutically acceptable salts, hydrates, and solvates thereof comprising:
i) contacting a flavonoid compound of formula (I), 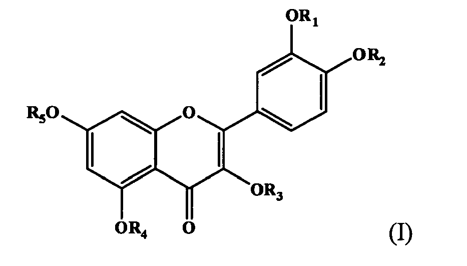 wherein R₁, R₂, R₃, R₄ and R₅, identical or different, are independently selected from H, benzyl, 3-(2,6-dimethyl-morpholin-4-yl)-2-hydroxy-1-propyl or combination thereof with carboxylic acid of general formula (V),

   R₆-(CH=CH)ₙ-CO₂H (V),

   wherein R₆ is linear or branched substituted or unsubstituted alkyl C₁ to C₁₆, or alkenyl C₂ to C₁₆, cyclic alkyl radical having from 5 to 6 carbon atoms, wherein cyclic alkyl radical is independently mono-, di-, tri, tetra substituted in any position on the ring with OH, O-alkyl having saturated or unsaturated, linear, branched or cyclic hydrocarbon chains having from 1 to 10 carbon atoms, acetyl, or combination thereof, 1-adamantyl, aryl, wherein aryl is independently mono-, di-, tri substituted in any position on the ring with OH, O-alkyl having saturated or unsaturated, linear, branched or cyclic hydrocarbon chains having from 1 to 10 carbon atoms, acetyl, or combination thereof and n is 0 or 1;
ii) undergoing debenzylation of benzylated flavonoid as obtained in the previous step i) using catalytic hydrogenation process.

Another object of the present invention is a process for the preparation of the dimer of compound of structural chemical formula (I) having general formula (II), which comprises contacting a flavonoid compound of general formula (I), with the proviso that the substitutent R₁ and R₂ is hydrogen with a metal salt at a temperature above 50°C in a solvent.

Another object of the present invention is to provide processes for preparing a pharmaceutical formulation which comprises the flavonoid compound of the general formula (I) or the dimer of formula (II) thereof in admixture with a pharmaceutically acceptable carrier.

In a still further aspect, the present invention provides novel quercetin derivatives of formula (I) and pharmaceutically acceptable salts, hydrates and solvates thereof or a dimer thereof for the preparation of a medicament for the treatment of photodermatitis, inflammation and allergy.

### DETAILED DESCRIPTION OF THE INVENTION

As mentioned hereinbefore, the present invention provides novel quercetin derivatives of formula (I) and pharmaceutically acceptable salts, hydrates, and solvates thereof, 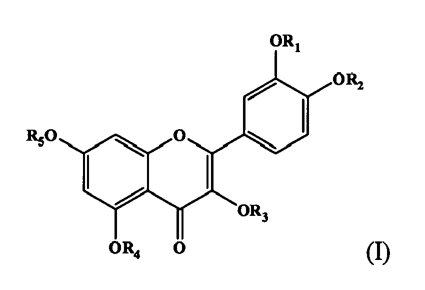 in which groups R₁ to R₅, identical or different, are independently selected from H, benzyl, 3-(2,6-dimethyl-morpholin-4-yl)-2-hydroxy-1-propyl, or a group R₆-(CH=CH)ₙ-CO- wherein R₆ is linear or branched substituted or unsubstituted alkyl C₁ to C₁₆, or alkenyl C₂ to C₁₆, cyclic alkyl radical having from 5 to 6 carbon atoms, wherein cyclic alkyl radical is independently mono-, di-, tri, tetra substituted in any position on the ring with OH, O-alkyl having saturated or unsaturated, linear, branched or cyclic hydrocarbon chains having from 1 to 10 carbon atoms, acetyl, or combination thereof, 1-adamantyl, aryl, wherein aryl is independently mono-, di-, tri substituted in any position on the ring with OH, O-alkyl having saturated or unsaturated, linear, branched or cyclic hydrocarbon chains having from 1 to 10 carbon atoms, acetyl, or combination thereof and n is 0 or 1; with the proviso that if in the group R₆-(CH=CH)ₙ-CO- substituent R₆ is phenyl and n is 0, hydroxyl group is not in ortho position or if R₆ is phenyl and n is 1, methoxy group is not in para position, and
a dimer of the compound of general formula (I) wherein R₁ to R₅ have the meanings given for general formula (I); and the isomers of the compound of general formula (I) and its dimer.

The representative compounds of formula I indicate the following derivatives summarized in Table I.

**Table I.**

| **R₁** | **R₂** | **R₃** | **R₄** | **R₅** |
|---|---|---|---|---|
| Ac | H | H | DMMP | H |
| Ac | Ac | Ac | DMMP | Ac |
| Piv | Piv | Piv | DMMP | Piv |
| palmitoyl | H | H | DMMP | H |
| oleoyl | H | H | DMMP | H |
| DMMP | Ac | Ac | DMMP | Ac |
| veratroyl | H | H | H- | H |
| H- | H | H | veratroyl | H |
| veratroyl | H | H | veratroyl | H |
| 3,4-di-O-acetylbenzoyl | H | H | H | H |
| 3,4-di-O-acetylbenzoyl | H | H | 3,4-di-O-acetylbenzoyl | H |
| 3,4- di-O-prenylbenzoyl | H | H | 3,4-di-O-prenylbenzoyl | H |
| 3,4- di-O-prenylbenzoyl | H | H | H | H |
| 2,5-di-O-prenylbenzoyl | H | H | 2,5-di-O-prenylbenzoyl | H |
| 3,4,5-tri-O-prenylbenzoyl | H | H | H- | H |
| 3,4,5-tri-O-prenylbenzoyl | H | H | 3,4,5-tri-O-prenylbenzoyl | H |
| H | H | H | 3,4,5-tri-O-prenylbenzoyl | H |
| H | H | H | di-O-prenylcaffeoyl | H |
| di-O-prenylcaffeoyl | Ac | Ac | Ac | Ac |
| di-O-prenylcaffeoyl | Bz | H | di-O-prenylcaffeoyl | H |
| di-O-prenylcaffeoyl | H | H | H- | H |
| di-O-prenylcaffeoyl | Bz | H | Bz | H |
| H | H | H | di-O-geranylcaffeoyl | H |
| di-O-geranylcaffeoyl | H | H | H- | H |
| di-O-geranylcaffeoyl | H | H | di-O-geranylcaffeoyl | H |
| Bz | H | H | di-O-diacetylcaffeoyl | H |
| di-O-geranylcaffeoyl | Bz | Bz | H- | Bz |
| di-O-geranylcaffeoyl | H | H | DMMP | H |
| di-O-diacetylcaffeoyl | H | H | di-O-diacetylcaffeoyl | H |

The following abbreviations were used: Ac (acetyl), Bz (benzyl), DMMP (3-(2,6-dimethyl-morpholin-4-yl)-2-hydroxy-1-propyl), Geranyl (*trans*-3,7-dimethyl-2,6-octadienyl), Piv (pivaloyl), Prenyl (3-methyl-2-butenyl)

Further, as mentioned hereinbefore, the present invention provides a process for preparation of the novel quercetin derivatives of formula (I), and pharmaceutically acceptable salts, hydrates, and solvates thereof comprising contacting a flavonoid compound of formula (I) 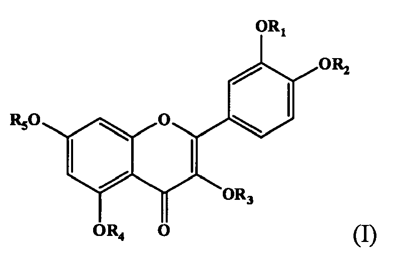 wherein R₁, R₂, R₃, R₄ and R₅, identical or different, are independently selected from H, benzyl, 3-(2,6-dimethyl-morpholin-4-yl)-2-hydroxy-1-propyl or combination thereof with an carboxylic acid of general formula (V),

R₆-(CH=CH)ₙ-CO₂H (V),

wherein R₆ is linear or branched substituted or unsubstituted alkyl C₁ to C₁₆, or alkenyl C₂ to C₁₆, cyclic alkyl radical having from 5 to 6 carbon atoms, wherein cyclic alkyl radical is independently mono-, di-, tri, tetra substituted in any position on the ring with OH, O-alkyl having saturated or unsaturated, linear, branched or cyclic hydrocarbon chains having from 1 to 10 carbon atoms, acetyl, or combination thereof, 1-adamantyl, aryl, wherein aryl is independently mono-, di-, tri substituted in any position on the ring with OH, O-alkyl having saturated or unsaturated, linear, branched or cyclic hydrocarbon chains having from 1 to 10 carbon atoms, acetyl, or combination thereof and n is 0 or 1. The conditions under which such acylation reactions with e.g. acyl halogenide are carried out are well known to those skilled in the art; they can be carried in the presence of a base and in the presence of a suitable solvent or alternatively in the presence of an activated carboxylic acid reagent. The acylation reaction can be carried out in conventional manner in the presence of a solvent to allow solubilization of the starting polyphenolic or selectively benzylated polyphenolic compounds. This acylation can be carried out on one or more or even the entire hydroxyl groups of quercetin or benzylated quercetin derivatives. The quercetin skeleton selectively benzylated in various positions may be obtained by known method (Tetrahedron, 2002, 58, 10001-10009). To allow selective derivatization the acetylation/benzylation strategy was adapted (J. Amer. Chem. Soc. 1958, 20, 5531, J. Amer. Chem. Soc.1958, 20, 5527, J. Org. Chem. 1962, 27, 1294). This alkylating method is also used for simple preparation of 3'- or 5- (2,6-dimethyl-morpholin-4-yl)-2-hydroxy-1-propyl substituted quercetin. For pentaesters derivatives and tetraester derivatives acylation was carried out by modifying the known procedure (J. Med. Chem. 2007, 50,241). The different stages of regioselective substitution of hydroxyls of quercetin in the processes are illustrated in examples of invention.

The acylating agent, the carboxylic acid derivatives of formula (V), are employed in stoichiometric proportions to excess the stoichiometric proportions of the of the flavonoid compound of formula (I). Typically, the carboxylic acid derivatives of formula (V) are employed in proportions of between 1 to 5 moles per mole of the flavonoid compound of formula (I). It is well-known that the nonequivalent hydroxyl functions of quercetin lead to the mixture of isomers with different proportion, thus unreacted quercetin is also detected in this type reaction simultaneously with various substituted products.

The acylating agent can advantageously be selected from acids of the formula R₆-(CH=CH)ₙ-CO₂H and the derivatives of such acids, particularly the acid halides of the formula R₆-(CH=CH)ₙ-COHal or the anhydrides of the formula (R₆-(CH=CH)n-CO)₂O.

A non-exhaustive list of carboxylic acid suitable for forming such derivatives is given: palmitic acid, ferulic acid, caffeic acid, 1-adamantane carboxylic acid, salicylic acid, cinnamic acid, gallic acid, quinic acid, chlorogenic acid. The compounds of the formula V or suitable derivatives thereof are commercial material or may be prepared by method known per se to the person skilled in the art and are described in standard works such as Houben-Weyl, "Methoden der organischen Chemie", Georg Thieme Verlag, Stuttgart.

If a carboxylic acid is used as the acylating agent, the reaction can be carried out in the presence of an activating agent for said acid, said activating agent normally being selectable from dicyclohexylcarbodiimide and tert-butyl chloroformate, which makes it possible to form a mixed anhydride.

In a exemplified embodiment, the flavonoid compound of formula (I), wherein R₂, R₃, R₄, R₅ is benzyl and R₁ is hydrogen reacted with a carboxylic acid derivatives of formula (V), in the presence of a base and in the presence of a activating agent in a suitable solvent at a temperature of between -10 to 120°C to produce the corresponding compounds of formula (I) wherein R₁ corresponding to R₆-(CH=CH)ₙ-CO- and R₂, R₃, R₄, R₅ is benzyl. The acylation reaction is complete in about 4 to 12 hours. The compounds of formula (I) is consequently isolated from the reaction mixture by suitable methods. Suitable solvents that can be employed for the reaction of the flavonoid compound of formula (I) and the carboxylic acid derivatives of formula (V) are preferably aprotic in nature and such aprotic solvents that can be employed include acetone, tetrahydrofurane, N,N-dimethylformamide, N,N-dimethylacetamide, dioxane, acetonitrile, dichloromethane, dichloroethane and the like, of which N,N-dimethylformamide or acetone is the preferred aprotic solvent.

Both organic and inorganic bases can be employed for the reaction of the flavonoid compound of formula (I) and the carboxylic acid derivatives of formula (V).

The organic bases that can be employed include tertiary amines like alkyl amines, pyridine, 2,6-lutidine, N-methyl-morpholine, 4-dimethylaminopyridine. Alkyl amines are preferred and amongst such alkyl amines, triethyl amine, di-isopropyl ethyl amines are preferred.

The inorganic bases that can be employed include alkali metal carbonates, such as sodium carbonate, potassium carbonate, lithium carbonate; and alkali metal bicarbonates, such as, sodium bicarbonate or potassium bicarbonate. Alkali metal carbonates are more preferred and amongst the alkali metal carbonates, potassium carbonate is preferred.

Upon completion of the reaction the compound of formula (I) can be isolated by suitable methods e.g. evaporation, precipitation, extraction, or chromatography. When water-miscible aprotic solvents like acetone, tetrahydrofurane and the like are employed in the reaction, the compound of formula (I) can be isolated by filtration of the reaction mixture whereas hydrochloride or hydrobromide salt of organic base is separated. When water-immiscible aprotic solvents like dichloromethane, dichloroethane and the like are employed in the reaction, the compound of formula (I) can be isolated by dilution of the reaction mixture with water, followed by separation of the organic and aqueous phases and isolation of the compound of formula (I) from the water-immiscible organic solvent by evaporation of the solvent or crystallization of the product from the same or precipitation of the product from the same by addition of a co-solvent. On the other hand, it is well-known the alternative strategies using the direct acylation of quercetin which depends on the relative reactivity of the different hydroxyl position toward an acylating agent gives complex reaction mixture. Therefore this straightforward path may be also occasionally applied.

Compounds of formula (I) wherein at least one from R₁ to R₅ is hydrogen are obtained by catalytic hydrogenation of the compounds of formula (I), in which at least one from R₁ to R₅ is benzyl.

The conditions under which such debenzylation reactions are carried out are well known to those skilled in the art. In general, the catalytic hydrogenation is carried out by subjecting the compounds of formula (I), to hydrogen pressure in the presence of an organic solvent and a hydrogenation catalyst at a hydrogen pressure from about 135 to 1380 kPa and at a temperature of from about 20°C to 60°C.

Typical hydrogenation catalysts that can be employed are selected from those of palladium or platinum supported on carbon. Suitable organic solvents that can be employed for the catalytic hydrogenation reaction are those selected from the class of organic acids, cycloethers, alcohols and mixtures thereof. Amongst the preferred organic solvents are those belonging to the class of cycloethers and alcohols and the preferred solvents are tetrahydrofuran and methanol.

After removal of the hydrogenation catalyst from the reaction mixture, the compound of formula (I) can be isolated in aforementioned manner depending on the solvents utilized for their preparation, isolation, and crystallization and may further be isolated as hydrates and solvates and such hydrates and solvates are construed to be within the scope and spirit of the present invention.

The biflavonoid is a dimer of two covalently bonded moieties which is each of general formula (I) as set out above. Bonding between the two moieties generally occurs at the 2, 3-position of one moiety and the 3', 4'-position of the other moiety. The preferred dimer has general formula (II) wherein R₁ to R₅ have the same meanings as for general formula (I). Known process for preparation of compound of general formula (II) wherein R₁ to R₅ is hydrogen, the quercetin dimer, is carried out in low yield by means of a large excess of peroxyl radical generator e.g. AIBN. This process results to a complex mixture that is very difficult to isolate from one another and purify. Applicant has now found that by simple reacting one mole of a flavonoid compound of the general formula (I) with 1 to 20 moles of the metal salt at a temperature above 50°C, one obtained practically with quantitative yield the dimer of the general formula (II). Typical dimerisation metal salt that can be employed are selected from those of silver, cooper, lead, cerium and iron salts such as carbonates, nitrate, sulfate, chloride, acetate or hydroxides and oxides. The metal salt (or a mixture of two salts) is introduced directly into the reactor in the form of a powder or as a suspension. The dimerisation reaction can be carried out in organic solvent or solvent mixture such as acetone, tetrahydrofurane, N,N-dimethylformamide, methanol, ethanol, dioxane, acetonitrile, benzene, toluene, dichloroethane and the like, of which toluene or acetone are preferred. In the process according to the invention quercetine dimer and new dimer flavonoid derivatives can be obtained in practically pure form and total yield.

Representative pharmaceutically acceptable salts of the novel quercetin derivatives of formula (I) wherein R₁ is 3-(2,6-dimethyl-morpholin-4-yl)-2-hydroxy-1-propyl as well as compounds of formula (I), wherein R₄ is 3-(2,6-dimethyl-morpholin-4-yl)-2-hydroxy-1-propyl include but are not limited to those salts such as fumarate, gluconate, glutamate, hydrochloride, hydrogen sulfate. The above-mentioned salts of the novel quercetin derivatives of formula (I) can be prepared by employing usual methods known in the art.

In yet another aspect, the present invention provides pharmaceutical compositions comprising a therapeutically effective amount of the novel quercetin derivatives of formula (I) and pharmaceutically acceptable salts, hydrates and solvates thereof that are useful for the treatment of various disorders including photodermatitis, inflammation and allergy.

In a further aspect the present invention relates to the use of novel quercetin derivatives of formula (I), their dimers and pharmaceutically acceptable salts, hydrates and solvates thereof in the preparation of a medicament for treating disorders including photodermatitis, inflammation and allergy.

Pharmaceutical compositions of the present invention contain the novel quercetin derivatives of general formula (I) or dimers of formula (II) and excipients. By virtue of the liposoluble property of quercetin derivatives according to the invention, these acylated quercetin derivatives can easily be incorporated into conventional cosmetic formulations, especially those in the form of creams, ointments, emulsions, gels or lotions.

According to the invention, the above-mentioned acylated quercetin derivatives can also be used in dietetics or in agri-foodstuff compositions.

The acylated flavonoids according to the invention make it possible to improve the stability of foodstuffs by virtue of their free radical inhibiting and antioxidizing activity. Agri-foodstuff compositions that may be mentioned are drinks, fruit juices, tonic drinks and dairy products.

In the formulation excipients are added to the composition for variety of purposes. Diluents (as for example microcrystalline cellulose, talc, calcium carbonate, magnesium oxide) increased the bulk of solid pharmaceutical composition, and may make a pharmaceutical dosage form containing the composition superior and giving care for patient. Binders for solid pharmaceutical compositions may include dextrin, gelatin, povidone, maltodextrin. Disintegrants may include colloidal silicon dioxide, crospovidone, and starch. Glidants also can be added to improve the flowability of a non-compacted solid composition and improve the accuracy of dosing. Selection of excipients and the amounts used may be readily determined by the formulation scientist based upon experience and consideration of standard procedures and references works in the field the art. Dosage forms of the present invention may include solid dosage forms such as for example tablets, pills, powders, granules and capsules. Liquid dosage forms may include liquid syrups, suspensions, emulsions, solutions and elixirs. Dosage forms of the present invention may include parenteral administration such as for example sterile aqueous, aqueous-organic and organic solutions, suspensions and emulsions. Topical administration or aerosol inhalation may include water, alcohol, glycol, and oil solution or oil-water emulsion. Rectal administration may include suppositories. The active ingredient and excipients may be formulated into compositions and dosage forms according to methods known in the art.

The invention is illustrated by reference to the following examples. However, the examples are not intended to limit any scope of the claims anyway. It will be apparent to those skilled in the art that many modifications, both to materials and methods, may be practiced without departing from the purpose and interest of this invention.

### EXAMPLES

### Example 1

### 3,7-dihydroxy-2-[4-hydroxy-3-(2,6-cis-dimethylmorpholin-4-yl-2-hydroxy-propyl)oxyphenyl]-5-hydroxy-chromen-4-one hydrochloride

### Step A

### Synthesis of 3,7-bis-benzyloxy-2-(4-benzyloxy-3-hydroxy-phenyl)-5-hydroxy-chromen-4-one and 3,7-bis-benzyloxy-2-(3,4-bisbenzyloxy-3-hydroxy-phenyl)-5-hydroxy-chromen-4-one

Benzyl bromide (55.6 g) was added to a mixture of quercetin (31.8 g) and potassium carbonate (52.5 g) in DMF (500ml). Reaction mixture was stirred for 12 h at 25 °C. The reaction mixture was diluted with water (1.2 1) and ethyl acetate (250 ml) was added. The organic layer was separated, washed with water, dried over anhydrous magnesium sulphate and evaporated to give crude product. The crude products were separated by column chromatography using ethyl acetate/hexane (1:1.5 v/v) as eluent to furnish 3,7-bis-benzyloxy-2-(4-benzyloxy-3-hydroxy-phenyl)-5-hydroxy-chromen-4-one, 19.5 g, m.p.=151-152 °C and 3,7-bis-benzyloxy-2-(3,4-bisbenzyloxy-3-hydroxy-phenyl)-5-hydroxy-chromen-4-one 22.8 g, m.p.=128-132 °C.

### Step B

### Synthesis of 3,7-Bis-benzyloxy-2-(4-benzyloxy-3-(2,3-epoxy-propoxy)phenyl)-5-hydroxy-chromen-4-one

Epichlorhydrine (0.44 g) was added to a mixture of 3,7-bis-benzyloxy-2-(4-benzyloxy-3-hydroxy-phenyl)-5-hydroxy-chromen-4-one (2.3 g) and potassium carbonate (2.5 g) in dry DMF (50 ml). Reaction mixture was stirred for 72 h at 25 °C under nitrogen atmosphere. The reaction mixture was diluted with water (120 ml) and ethyl acetate (120 ml) was added. The organic layer was separated, washed with water, dried over anhydrous magnesium sulphate and evaporated to give crude product. The crude product was purified by column chromatography using ethyl acetate/hexane (1:1.5 v/v) as eluent to furnish the title product as solidified oil. Yield 2.4 g, MS pseudomolecular ion 629 m/z.

### Step C

### Synthesis of 3,7-Bis-benzyloxy-2-[4-benzyloxy-3-((2,6-cis-dimethylmorpholin-4-yl)-2-hydroxy-propoxy)phenyl]-5-hydroxy-chromen-4-one

3,7-Bis-benzyloxy-2-(4-benzyloxy-3-(2,3-epoxy-propoxy)phenyl)-5-hydroxy-chromen-4-one (1.4 g) and 2,6-cis-dimethylmorpholine (0.38 g) was refluxed in mixture of tetrahydrofurane/chloroform (20 ml, 1:1 v/v) for 12 h. The resultant cloudy mixture was filtered over Celite. The filtrate was evaporated to afford crude product. The product was purified by column chromatography using methylene chloride/methanol (9:0.5, v/v) as eluent to furnish the title product. Yield 0.85 g, m.p. 105-110 °C.

### Step D

### Synthesis of 3,7-dihydroxy-2-[4-hydroxy-3-(2,6-cis-dimethylmorpholin-4-yl-2-hydroxy-pro-poxy)phenyl]-5-hydroxy-chromen-4-one hydrochloride

To a solution of 3,7-dihydroxy-2-[4-hydroxy-3-(2,6-cis-dimethylmorpholin-4-yl-2-hydroxy-propoxy)phenyl]-5-hydroxy-chromen-4-one (0.4 g) in tetrahydrofurane/methanol (20 ml, 1:1, v/v) was added 10% Pd/C on charcoal (0.04 g, 10% w/w). The resulting mixture was shaken under a hydrogen atmosphere (345 kPa) for 12 hours at 25 °C. The resultant mixture was filtered over Celite. The filtrate was evaporated to afford crude product. The crude product was purified by column chromatography using methylene chloride/Methanol (9:0.5, v/v) as eluent to furnish the solidified oily material. Yield 0.25g, MS pseudomolecular ion 472 m/z. The solidified material was dissolved in 5 ml of ethanol and hydrochloric acid in diethyl ether was added at 0 °C. Precipitated white material was filtered to give 0.2 g 3,7-dihydroxy-2-[4-hydroxy-3-(2,6-cis-dimethylmorpholin-4-yl-2-hydroxy-propoxy)phenyl]-5-hydroxy-chromen-4-one hydrochloride. The compound was isolated as ethanol solvate as seen in ¹H NMR and TGA.

### Example 2

### Synthesis of 3,7-dihydroxy-2-[3,4-dihydroxy-phenyl]-5-(2,6-cis-dimethylmorpholin-4-yl-2-hydroxy-propoxy)chromen-4-one

Analogous to example 1, but 3,7-bis-benzyloxy-2-(3,4-bisbenzyloxyphenyl)-5-hydroxy-chromen-4-one (0.55 g) from example 1 step A, was used.

Yield 0.25 g.

### Example 3

### Synthesis of 3,7-bis-(2-acetylsalicyloyloxy)-2-(3,4-bis-(2-acetylsalicyloyloxy)phenyl)-5-hydroxy-chromen-4-one

2-Acetylsalicyloyl chloride (1.6 g) in acetone (20 ml) was added to a mixture of quercetin (0.6 g) and triethylamine (0.8 g) in dry acetone (50 ml). Reaction mixture was stirred for 12 h at 25 °C under nitrogen atmosphere. The reaction mixture was filtered and evaporated to dryness to give crude product. The crude product was purified by column chromatography using ethyl acetate/hexane (1:1.5 v/v) as eluent to furnish the title product as solidified oil. Yield 1.6 g, m.p.= 102-104°C. MS pseudomolecular ion 949 m/z.

### Example 4

### Synthesis of 3-(O,O,O-trimethyl galloyloxy)-2-(3,4-bis-(O,O,O-trimethyl galloyloxy)phenyl)-7,5-dihydroxy-chromen-4-one and 3-(O,O,O-trimethyl galloyloxy)-2-(3,4-bis-(O,O,O-trimethyl galloyloxy)phenyl)-7-(O,O,O-trimethyl galloyloxy)-5-hydroxy-chromen-4-one

O,O,O-Trimethyl galloyl chloride (1.15 g) in acetone (20 ml) was added to a mixture of quercetin (0.3 g) and triethylamine (0.55 g) in dry acetone (50 ml). Reaction mixture stirred for 6 h at 0 °C and then 2h at 25 °C. The reaction mixture was filtered and evaporated to dryness to give crude product. The product was separated from crude mixture (monitored by LC/Mass) of tetra and trisubstitited quercetin derivative and unreacted quercetin by column chromatography using ethyl acetate/hexane (1:1.5 v/v) as eluent. The title compound 3-(O,O,O-trimethyl galloyloxy)-2-(3,4-bis-(O,O,O-trimethyl galloyloxy)phenyl)-7,5-dihydroxy-chromen-4-one was isolated as solidified oil, 0.75 g, m.p.= 160-161 °C. MS pseudomolecular ion 883 m/z. Further elution give (0.2 g) 3-(O,O,O-trimethyl galloyloxy)-2-(3,4-bis-(O,O,O-trimethyl galloyloxy)phenyl)-7-(O,O,O-trimethyl galloyloxy)-5-hydroxy-chromen-4-one.

### Example 5

### Synthesis of 1,3,11a-trihydroxy-9-(3,5,7-trihydroxy-4H-1-benzopyran-4-on-2-yl)-5a-(3,4-dihydroxyphenyl)-5,6,11-hexahydro-5,6,11-trioxanaphtacene-12-one

Silver carbonate (3 g) was added to a suspension of quercetin (3.1 g) in benzene: acetone (1:1) (200 ml). The resulting mixture was stirred under reflux for 6hours. The resultant mixture was filtered on Celit. Filtrate was evaporated to afford crude product as mixture diquercetin and triquercetin oligomers 85:15 (by HPLC/MS). The crude product was purified by crystallization from ethanol/acetone (filtering on milipores) to give the title compound as slightly brown solid (2.7 g), m.p.220-225 °C (Kofler bench). ¹H NMR (DMSO-*d*₆, 500 MHz): 2.07 (s, OH), 5.97 (bs, 2H, H-6, H-8), 6.19 (d, 1H, *J*) 2 Hz, H8/H6), 6.48 (d, 1H, *J*) 2 Hz, H-6/H8), 6.68 (dd, *J*) 8.6 Hz, 2.5 Hz, 1H, H-6Ar), 6.92 (d, *J*) 8.6 Hz, 1H, H-5Ar), 7.14 (bs, 1H, H-2Ar), 7.26 (d, *J*) 9 Hz, 1H, H-5Ar), 7.79 (d, *J*) 2 Hz, 1H, H-2Ar), 7.85 (dd, *J*) 8.5 Hz, 2 Hz, 1H, H-6Ar), 8.9(s, OH), 9.2 (s, OH), 9.29 (s, OH), 9.8 (s, OH), 10.85 (s, OH), 11.2 (s, OH), 12.36 (s, OH). MS pseudomolecular ion 601 m/z.

### Example 6

Analogous to example 5, but as the catalyst PbOAc₄ was used.

### Example 7

### Synthesis of 1,3-bis-butyryloxy-11a-hydroxy-9-(3,5,7-tributyryloxy-4H-1-benzopyran-4-on-2-yl)-5a-(3,4-bisbutyryloxyphenyl)-5,6,11-hexahydro-5,6,11-trioxanaphtacene-12-one

Butyric anhydride (2 ml) and product from example 5 (0.37 g) was stirred at 80°C for 6hours. The reaction mixture was diluted with water (50 ml) and then ice (10 g) was added with vigorous stirring. The precipitated product was purified crystallization from ethanol/acetone afforded the title compound (0.53 g), as slightly yellowish solid, m.p. 129-135 °C. MS pseudomolecular ion 1091 m/z.

### Example 8

### Synthesis of 3-(O-prenylferuloyloxy)-2-(3,4-bis-(O-prenylferuloyloxy)phenyl)-7-(O-prenyl-feruloyloxy)-5-hydroxy-chromen-4-one

O-Prenylferuloyl chloride (2.8 g) in acetone (30 ml) was added to a mixture of quercetin (0.75 g) and triethylamine (1.1 g) in dry acetone (50 ml). Reaction mixture stirred for 12 h at 25 °C under nitrogen atmosphere. The reaction mixture was filtered and evaporated to dryness to give crude product. The product was separated from crude mixture (monitored by HPLC/MS) of tetra and triacylated quercetin by column chromatography using ethyl acetate/hexane (1:1.5 v/v) as eluent. The required product crystallized as off white solid from appropriate chromatographic fraction. Yield 1.1 g.

### Example 9

### Step A

### Synthesis of 1,3,4,5-tetraacetoxycyclohexane carboxylic acid

To a suspension of D-(-)-quinic acid (2 g) in a 2 : 1 mixture of acetic acid-acetic anhydride (15 mL) one drop of perchloric acid was added at room temperature. As the reaction temperature increased to 50-60 °C, the mixture became clear. The solution was stirred for a further 12 h, diluted with chloroform and then successively extracted with saturated aq.NaHCO₃ and water. The organic layer was dried over Na₂SO₄, filtered and concentrated under reduced pressure. The residue was finally precipitated by addition of hexane to give 1,3,4,5-tetraacetoxycyclohexane carboxylic acid (3 g).

### Step B

### Synthesis of 3-(1,3,4,5-tetraacetoxycyclohexane carbonyl)oxy-2-(3,4-bis-(1,3,4,5-tetraacet-oxycyclohexanecarbonyloxy)phenyl)-7,5-dihydroxy-chromen-4-one and 3-(1,3,4,5-tetraacetoxycyclohexane carbonyl)oxy-2-(3,4-bis-(1,3,4,5-tetraacetoxycyclohexa-necarbonyloxy)phenyl)-5-hydroxy-7-(1,3,4,5-tetraacetoxy-cyclohexane carbonyl)oxy-chro-men-4-one

1,3,4,5-Tetraacetoxycyclohexanecarbonyl chloride prepared from 1,3,4,5-tetraacetoxycyclohexane carboxylic acid, example 9 step A (1.0 g) and thionylchloride (1.2 g) was dissolved in dichloromethane (50 ml). Yellowish solution was added to a mixture of quercetin (0.84 g) and triethylamine (1.1 g) in dichloromethane (50ml). Reaction mixture was stirred for 12 h at 25 °C under nitrogen atmosphere. The reaction mixture was diluted with water (50 ml) and the organic layer was successively extracted with saturated aq.NaHCO₃ and water. The organic layer was dried over Na₂SO₄, filtered and concentrated under reduced pressure. The organic layer was separated, washed with water, dried over anhydrous sodium sulphate and evaporated to give crude product. The product was separated from crude mixture of tetra : tri substituted flavonoid derivative and unreacted quercetine (monitored by HPLC/MS). The column chromatography used ethyl acetate/hexane (1:1.5 v/v) as eluent to furnish 3-(1,3,4,5-tetraacetoxycyclohexane carbonyloxy)-2-(3,4-bis-(1,3,4,5-tetraacetoxycyclohexanecarbonyl-oxy)phenyl)-7,5-dihydroxy-chromen-4-one 0.32 g and 3-(1,3,4,5-tetraacetoxycyclohexane carbonyloxy)-2-(3,4-bis-(1,3,4,5-tetraacetoxycyclohexanecarbonyloxy)phenyl)-5-(1,3,4,5-tet-raacetoxycyclohexane carbonyloxy)-7-hydroxy-chromen-4-one 0.12 g.

### Example 10

### Synthesis of 3,7-bis-hydroxy-2-(4-hydroxy-3-(chloropivaloyloxy)phenyl)-5-hydroxy-chro-men-4-one

### Step A

Chloropivaloyl chloride (1.05 g) in acetone (30 ml) was added to a mixture of 3,7-bis-benzyloxy-2-(4-benzyloxy-3-hydroxy-phenyl)-5-hydroxy-chromen-4-one from example 1, step A (1.0 g) and triethylamine (0.69 g) in dry acetone (50 ml). Reaction mixture was stirred for 12 h at 25°C under nitrogen atmosphere. The reaction mixture was filtered and evaporated to dryness to give crude product. 3,7-Bis-benzyloxy-2-(4-benzyloxy-3-(chloropivaloyl-oxy)phenyl)-5-hydroxy-chromen-4-one was separated by column chromatography using ethyl acetate/hexane (1:1.5 v/v) as eluent. The required product crystallized as off white solid. Yield 1.1 g.

### Step B

To a solution of product from step A (0.8 g) in methanol (80 ml) was added 10% Pd/C on charcoal (0.08 g, 10% w/w). The resulting mixture was shaken under a hydrogen atmosphere (415kPa) for 12 hours at 25 °C. The resultant mixture was filtered over Celite. The filtrate was evaporated to afford crude product. The crude product was purified by column chromatography using methylene chloride/methanol (9:0.5, v/v) as eluent to furnish the title compound. Yield 0.2 g. The title compound was isolated as methanol solvate as seen in ¹H NMR and TGA.

### Example 11

The solubility of the novel quercetin derivatives of formula (I) and formula (II) were compared with quercetin as illustrated in the Table 1.

**Table 1. Comparison of the solubility of the novel quercetin derivatives of formula (I) and of formula (II) in water and ethanol at 25°C, quercetin = 1**

| Compound | water | ethanol |
|---|---|---|
| Example 1 | 8.8 | 1.1 |
| Example 3 | 1.7 | 10.5 |
| Example 7 | 1.1 | 12.8* |
| Example 10 | 1.0 | 11.3 |
| Formula (I), R₁=acetyl,R₂,R₃,R₅=H, R₄ = 2,6-cis-dimethylmorpholin-4-yl-2-hydroxy-propyl | 7.9 | 7.8 |
| Formula (II), R₁, R₂ = 2-acetylsalicyloyl, R₃-R₅=H | 1.2 | 15.8 |
| Formula (II), R₁, R₂, R₄, R₅=acetyl, R₃=H | 1.0 | 10.1 |

| | | |
|---|---|---|
| *acetone was used | | |

### Example 12

### Synthesis of 2-(3, 4-dihydroxyphenyl)- 3,5,7-tris-(O-prenylferuloyloxy)chromen-4-one

O-Prenylferuloyl chloride (4.6 g), synthesized analogically to the known procedure (Carbohydr. Res. 1992, 229, 183) in dry DMF (40 ml) was added to a mixture of 2-(2,2-diphenylbenzo[1,3]dioxol-5-yl)-3,5,7-trihydroxychromen-4-one (2.3 g) and triethylamine (2.5 ml) in dry DMF (50 ml) at temperature 0 °C. The reaction mixture was allowed to warm to 25 °C over 1 h and stirring was maintained for 12 h at 65 °C. The reaction mixture was filtered and the filtrate was evaporated to dryness under vacuum. The crude reaction mixture was separated by flash column chromatography using dichloromethane as eluent. After solvent evaporation, the residue (1.6 g) was added to a mixture of acetic acid /water (70 ml, 5:1) and refluxed for 2 h. Then water (50 ml) and dichloromethane (250 ml) were added. The organic layer was dried and solvent evaporated. The residue was purified by flash column chromatography using dichloromethane as eluent. The title product crystallized standing from appropriate chromatographic fraction. Yield 0.9 g.

### Example 13

Synthesis of 1, 11a-dihydroxy-3-benzyloxy-9-(3,5-dihydroxy-7-benzyloxy-4H-1-benzopyran-4-on-2-yl)-5a-(3,4-dihydroxyphenyl)-5,6,11-hexahydro-5,6,11-trioxanaphtacene-12-one Analogically to Example 5, but 7-O-benzylquercetin (0.15 g) synthesized according to the known procedure (J. Amer. Chem. Soc. 1958, 20, 5531) was used.

### Example 14

### Synthesis of octaacylated quercetin dimer

Palmitoyl chloride (1.6 g) is added dropwise to the product from example 5 (0.3 g) and pyridine (15 ml). The mixture is heated at 105 °C for 0.5 h. The viscous brown solution is concentrated under vacuum and crude product is dissolved in dichloromethane (30 ml) and water (20 ml). The organic layer is successively washed with 10% NaOH, dried over Na₂SO₄ and then concentrated. The crude product (0.42 g) is purified on a column of silica using dichloromethane as eluent to give 0.22 g of octaacylated quercetin dimer (off-white powder).

### Example 15

### Determination of the free radical inhibiting activity in Vitro

A 50 µM solution of 1,1-diphenyl-2-picrylhydrazyl (DPPH) in ethanol is added for 30 minutes at room temperature to a tested sample. The drop in absorbance is then measured and the results are given as the percentage decrease in OD caused by the test compound relative to the solvent used. The tested products are dissolved in ethanol (except product according to Example 7 when DMSO was used); the results (solvent control subtracted) are given as % free radical inhibiting activity as described in table 2.

**TABLE 2.**

| Compound at 25mM | Free radical inhibiting activity |
|---|---|
| Quercetin | 62% |
| Example 3 | 82% |
| Example 7 | 95% |
| Example 8 | 75% |
| Formula (II), R₁, R₂ = 2-acetylsalicyloyl, R₃-R₅=H | 85% |
| Formula (I), R₁= O,O-diprenylcaffeoyl, R₂-R₅=H | 92% |

## Claims

1. A compound of general formula (I) and pharmaceutically acceptable salts, hydrates, and solvates thereof, 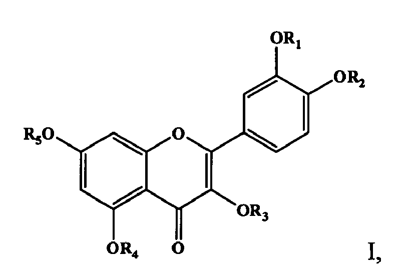 in which groups R₁ to R₅, identical or different, are independently selected from H, benzyl, 3-(2,6-dimethyl-morpholin-4-yl)-2-hydroxy-1-propyl, or a group R₆-(CH=CH)ₙ-CO- wherein R₆ is linear or branched substituted or unsubstituted alkyl C₁ to C₁₆ or alkenyl C₂ to C_{16,} cyclic alkyl radical having from 5 to 6 carbon atoms, wherein cyclic alkyl radical is independently mono-, di-, tri, tetra substituted in any position on the ring with OH, O-alkyl having saturated or unsaturated, linear, branched or cyclic hydrocarbon chains having from 1 to 10 carbon atoms, acetyl, or combination thereof, 1-adamantyl, aryl, wherein aryl is independently mono-, di-, tri substituted in any position on the ring with OH, O-alkyl having saturated or unsaturated, linear, branched or cyclic hydrocarbon chains having from 1 to 10 carbon atoms, acetyl, or combination thereof and n is 0 or 1; with the proviso that if in the group R₆-(CH=CH)ₙ-CO- substituent R₆ is phenyl and n is 0, hydroxyl group is not in ortho position or if R₆ is phenyl and n is 1, methoxy group is not in para position; and
a dimer of the compound of general formula (I) wherein R₁ to R₅ have the meanings given for general formula (I); and the isomers of the compound of general formula (I) and its dimer.

2. The compound according to claim 1 wherein the biflavonoid dimer has the general formula (II) 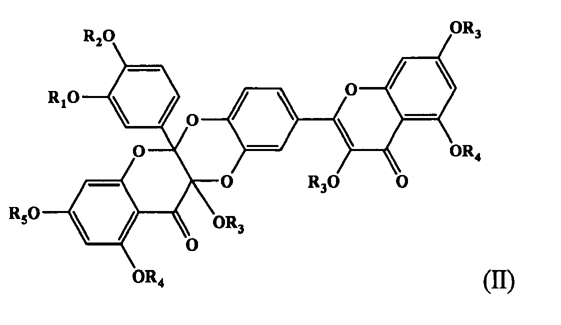 wherein R1 to R5 have the meanings given in claim 1, with the proviso that at least one of the substituent R₁ to R₅ is a hydrogen atom.

3. A process for preparation of compound of formula (I) and pharmaceutically acceptable salts, hydrates, and solvates thereof, comprising the steps of:
i) contacting of a flavonoid compound of formula (I), 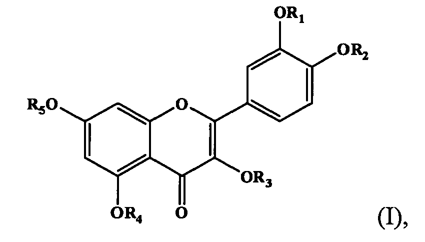 wherein R₁, R₂, R₃, R₄ and R₅, identical or different, are independently selected from H, benzyl, 3-(2,6-dimethyl-morpholin-4-yl)-2-hydroxy-1-propyl or from combination thereof with an carboxylic acid of general formula (V)
R₆-(CH=CH)ₙ-CO₂H (V),
wherein R₆ is linear or branched substituted or unsubstituted alkyl C₁ to C₁₆ or alkenyl C₂ to C₁₆, cyclic alkyl radical having from 5 to 6 carbon atoms, wherein cyclic alkyl radical is independently mono-, di-, tri, tetra substituted in any position on the ring with OH, O-alkyl having saturated or unsaturated, linear, branched or cyclic hydrocarbon chains having from 1 to 10 carbon atoms, acetyl, or combination thereof, 1-adamantyl, aryl, wherein aryl is independently mono-, di-, tri substituted in any position on the ring with OH, O-alkyl having saturated or unsaturated, linear, branched or cyclic hydrocarbon chains having from 1 to 10 carbon atoms, acetyl, or combination thereof and n is 0 or 1 and
ii) undergoing debenzylation of the compound of formula (I), as obtained in step (i), wherein at least one from R₁ to R₅ is benzyl, using catalytic hydrogenation to obtain compound of formula (I) wherein at least one from R₁ to R₅ is hydrogen with the proviso that at least one of the substituents R₁ to R₅ is a group R₆-(CH=CH)ₙ-CO-.

4. The process as claimed in claim 3, wherein carboxylic acid derivatives of general formula (V), are employed in proportions of between 1 to 5 moles per mole of the flavonoid compound of formula (I).

5. The process as claimed in claim 3, wherein the process is carried out in the presence of a base.

6. The process as claimed in claim 5, wherein the base is an organic or an inorganic base.

7. The process as claimed in claim 6 wherein said organic base is selected from tertiary amines such as alkyl amines, pyridine, and N-methyl-morpholine.

8. The process as claimed in any one of claims 3 to 7, wherein the reaction of the flavonoid compound of formula (I) and carboxylic acid derivatives of general formula (V) is carried out at a temperature of from about -10°C to about 120°C.

9. A process for preparation of compound of formula (II) as claimed in claim 2 which comprises contacting a flavonoid compound of general formula (I) as claimed in claim 1, with the proviso that that at least R₁ and R₂ is hydrogen, with a metal salt.

10. The process as claimed in claim 9 wherein the metal salt is selected from those of silver, cooper, lead, cerium and iron salts such as carbonates, sulfate, nitrate, chloride, acetate or hydroxides and oxides.

11. A pharmaceutical composition comprising the compound of formula (I) according to claim 1 or pharmaceutically acceptable salts, hydrates and solvates thereof or compound of formula (II) according to claim 2 and a compatible pharmaceutically acceptable carrier, adjuvant or diluent.

12. The pharmaceutical composition of claim 11 in the form of a unit dosage form for parenteral or oral administration.

13. Use of the compound of formula (I) as claimed in claim 1 or the compound of formula (II) as claimed in claim 2 in the preparation of a medicament for treating disorders including photodermatitis, inflammation and allergy.
